Europäisches Patentamt

European Patent Office

Office européen des brevets

(11) Numéro de publication: **0 289 382 B1**

## FASCICULE DE BREVET EUROPEEN

(45) Date de publication de fascicule du brevet: **30.12.92**  (51) Int. Cl.5: **G10K 11/34**

(21) Numéro de dépôt: **88400846.7**

(22) Date de dépôt: **08.04.88**

(54) **Générateur d'impulsions élastiques ayant une forme d'onde prédéterminée désirée et son application au traitement ou au diagnostic médical.**

(30) Priorité: **28.04.87 FR 8705980**

(43) Date de publication de la demande:
**02.11.88 Bulletin 88/44**

(45) Mention de la délivrance du brevet:
**30.12.92 Bulletin 92/53**

(84) Etats contractants désignés:
**AT BE CH DE GB IT LI NL SE**

(56) Documents cités:
EP-A- 0 068 961          EP-A- 0 072 498
DE-A- 3 119 295          US-A- 2 484 626
US-A- 3 879 698          US-A- 3 911 730
US-A- 3 924 259          US-A- 4 550 606

(73) Titulaire: **EDAP INTERNATIONAL
Z.I. le Parc aux Vignes Rue des Vieilles Vignes
F-77200 Croissy Beaubourg(FR)**

(72) Inventeur: **Dory, Jacques
91, rue des Molveaux
F-77450 Coupvray(FR)**

(74) Mandataire: **Marquer, Francis et al
Cabinet Moutard 35, Avenue Victor Hugo
F-78960 Voisins le Bretonneux(FR)**

Rank Xerox (UK) Business Services

## Description

Lorsque des impulsions élastiques, en particulier des impulsions élastiques de grande puissance telles que celles utilisées en lithotripsie extra-corporelle, se propagent dans un liquide de couplage, les pics négatifs que comporte inévitablement leur forme d'onde engendrent des variations de pression au sein du liquide et, lorsque la pression locale descend au-dessous de la pression de vapeur saturante, il peut se former des cavités remplies de vapeur au sein du liquide. Dès que ces bulles de vapeur atteignent des régions de plus forte pression, elles se condensent brutalement.

Le Déposant a découvert que ce phénomène de cavitation a tendance à freiner la propagation de tout pic positif précédé d'un pic négatif et, par conséquent, à réduire considérablement la puissance transmise aux cellules à traiter ou à examiner par un générateur d'impulsions élastiques et à entraîner diverses perturbations des effets recherchés dans l'application des impulsions élastiques à l'examen échographique ou au traitement par concentration de puissance ultra-acoustique en un point focal. En lithotripsie extra-corporelle, la cavitation présente l'inconvénient particulièrement important de provoquer une sensation de picotement qui peut atteindre le seuil de douleur.

La présente invention a pour objet un procédé de génération d'impulsions élastiques mettant en oeuvre un transducteur piézoélectrique ou magnétostrictif focalisant ou associé à des moyens de focalisation, ledit transducteur ayant une pluralité de surfaces émettrices élémentaires engendrant des signaux élastiques qui se superposent, après propagation sur une certaine distance, dans une région locale et des moyens d'exciter périodiquement à une cadence déterminée plusieurs groupes de surfaces émettrices élémentaires avec des impulsions électriques respectives distinctes, lesdites surfaces émettrices élémentaires appartenant à des éléments transducteurs électriquement isolés les uns des autres ayant des fonctions de transfert différentes, caractérisé en ce que lesdites fonctions de transfert correspondent à des fréquences de résonance multiples entiers de l'une d'entre elles et que des moyens sont prévus pour réaliser entre les signaux électriques respectifs qui se superposent dans la région focale, des déphasages non nuls prédéterminés, de façon à obtenir dans ladite région une forme d'onde globale fixée à l'avance.

Suivant un mode d'exécution préféré, lesdites fréquences de résonance sont F, 2F, 3F et 4F ou F, 2F et Fet les déphasages de chacun des signaux électriques par rapport à une référence de phase arbitraire commune sont égaux à $-5/(4F_n)$, $F_n$ étant la fréquence de résonance de l'un desdits éléments transducteurs.

Suivant une première forme d'exécution, le transducteur est constitué par un support en forme de calotte sphérique sur la surface de laquelle sont distribués des transducteurs piézoélectriques céramiques élémentaires d'épaisseurs inversement proportionnelles à leurs fréquences de résonance.

De préférence, les moyens d'excitation comportent des générateurs de fréquences différentes synchronisés par un même signal de synchronisation qui détermine ladite cadence d'émission et des lignes à retard réglables qui constituent lesdits moyens de déphasage.

Suivant une seconde forme d'exécution, le transducteur est constitué par une céramique monobloc usinée de façon à former des surfaces émissives élémentaires d'épaisseurs différentes électriquement isolées les unes des autres.

L'art antérieur est constitué par les documents DE-A-3 119 295 et EP-A-072 498.

Le premier de ces documents divulgue le déphasage des signaux d'excitation d'éléments transducteurs respectifs, ayant éventuellement des surfaces d'émission décalées les unes par rapport aux autres, en vue d'obtenir une focalisation électrique des ondes émises. Les signaux émis par les différents éléments arrivent en phase au foyer du transducteur.

Le second document enseigne de grouper en réseau des éléments transducteurs d'épaisseurs différentes, en vue d'améliorer la résolution à différentes distances du transducteur en agissant sur le diamètre du faisceau.

D'autres particularités, ainsi que les avantages de l'invention, apparaîtront clairement à la lumière de la description ci-après :
Au dessin annexé :
La figure 1 représente des formes d'ondes destinées à expliquer le procédé de l'invention ;
La figure 2 est une vue schématique en plan de la face d'émission d'un transducteur en forme de coupelle sphérique, dont
La figure 3 est une vue schématique en coupe par un plan de symétrie ;
La figure 4 est un schéma de circuit d'excitation ;
La figure 5 représente un ensemble transducteur plan usiné avec son circuit d'excitation ; et
La figure 6 est une vue partielle en coupe suivant VI-VI de la figure 5.

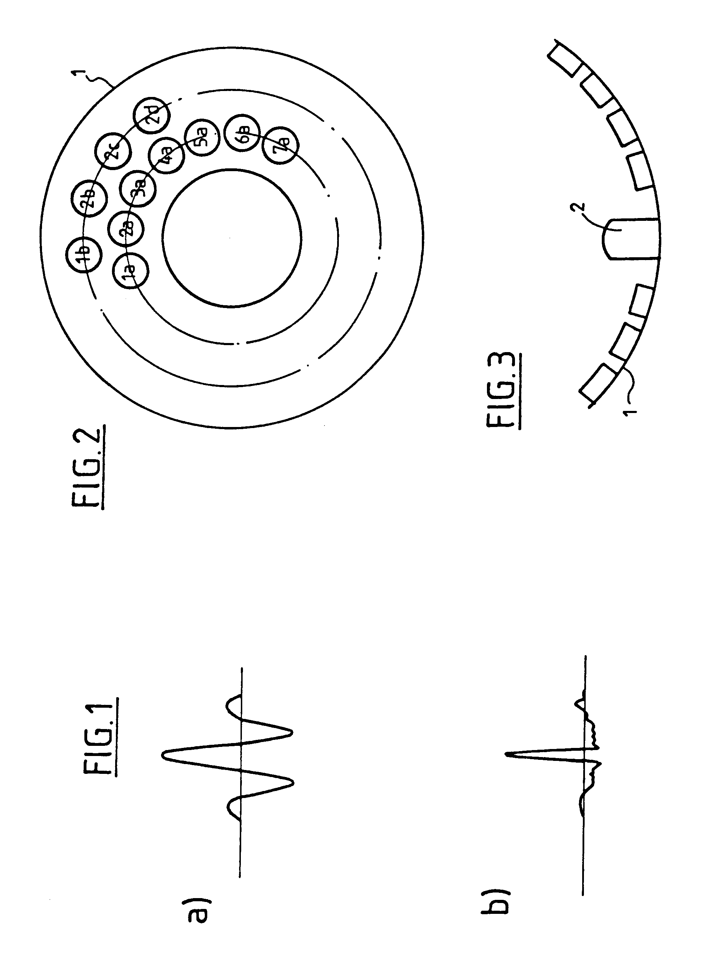

A la figure 1, on a représenté en (a) une impulsion élastique de courte durée produite au moyen d'un transducteur piézoélectrique tel qu'on les réalise actuellement, lorsqu'on veut engendrer une impulsion accompagnée du nombre minimum d'oscillations amorties, dont la face arrière a été convenablement amortie, tandis que des lames d'adaptation des impédances acoustiques ont été montées sur sa face avant. Une telle impulsion comporte des alternances de signes opposés de la haute fréquence d'excitation du transducteur, et la présence des alternances négatives entraîne une perte d'efficacité significative, due à la cavitation, dès que la puissance émise est relativement élevée (ce qui est le cas pour les sondes utilisées en thérapie).

On notera qu'il est indispensable que la polarité du transducteur soit choisie pour que le premier pic d'amplitude significative soit positif, car dans le cas contraire, la cavitation provoquée par un premier pic négatif empêcherait la propagation du reste du signal.

On constate à la figure 1(a) que ce premier pic a une amplitude sensiblement plus faible que celle du second : il en résulte finalement, d'une part, que toute la puissance du signal n'est pas exploitée, d'autre part, que la cavitation peut provoquer des effets nuisibles.

Il convient de souligner que même un transducteur théoriquement parfait, quelle que soit la forme de son signal d'excitation, engendrera au moins deux alternances de signes opposés, du fait qu'il comporte nécessairement deux surfaces émettrices séparées par une demi-longueur d'onde et émettant en opposition de phase.

En (b) on a représenté une impulsion élastique produite au moyen d'un transducteur piézoélectrique composé d'une pluralité d'éléments excités de manière indépendante dont les fréquences de résonance respectives sont $F_1$, $F_2$...$F_n$, ces éléments étant excités par des impulsions électriques de fréquences porteuses respectives $F_1$, $F_2$...$F_n$ respectivement déphasées par rapport à une origine des phases arbitraires de $-5/4F_1$, $-5/4F_2$, $-5/4F_n$. L'expérience montre que, si ces signaux électriques ont la même amplitude ou des amplitudes voisines et que les éléments du transducteur sont construits de façon à engendrer la forme d'onde acoustique représentée en (a), la superposition des signaux acoustiques ainsi engendrés donne la forme d'onde représentée en (b).

Dans cette forme d'onde, les alternances négatives ont une amplitude relative très réduite. L'expérience montre qu'il suffit de prendre par exemple quatre éléments ayant des fréquences F, 2F, 3F et 4F, ou même trois éléments ayant des fréquences F, 2F et 4F pour obtenir une prédominance suffisante de l'alternance positive unique.

Lorsque de tels éléments sont par exemple placés sur une calotte sphérique, leur trajet, jusqu'au centre de la sphère qui constitue le foyer, est identique et les signaux acoustiques arrivent donc au foyer avec les mêmes phases relatives qu'au moment de leur émission. On pourrait envisager de les placer sur une surface différente, par exemple plane, et il faudrait alors modifier les déphasages des signaux d'excitation pour obtenir une focalisation en tenant compte des différences de trajets entre les différents éléments.

Les éléments excités par des fréquences différentes déphasées entre elles pourraient être en nombre supérieur à quatre (ou répartis en plus de quatre groupes constitués chacun d'éléments excités par le même signal) et les fréquences et déphasages pourraient être différents de ceux qui ont été indiqués ci-dessus à titre d'exemple préféré.

L'un des objets de l'invention est la génération d'une forme d'onde élastique brève prédéterminée (en pratique, comportant avantageusement la prédominance d'un pic positif unique) par superposition de plusieurs formes d'ondes élastiques élémentaires. Cette génération est obtenue en superposant, dans une même région de l'espace, des ondes élastiques individuelles ayant au moins des caractéristiques de fréquence et de phase différentes qui se combinent entre elles pour donner la forme d'onde résultante souhaitée. En pratique, on effectuera, grâce à une commande assistée par ordinateur du réglage des paramètres fréquence, phase et même amplitude des formes d'ondes individuelles, une simulation de la forme d'onde résultante pour optimiser celle-ci en faisant varier lesdits paramètres.

On soulignera qu'il ne suffirait pas de faire varier les paramètres du signal électrique d'excitation d'un transducteur traditionnel ayant une surface d'émission homogène pour obtenir le résultat recherché, car la réponse impulsionnelle d'un tel transducteur serait au mieux du type de celle de la figure 1(a).

Par contre, avec un transducteur hétérogène du type décrit, on pourrait envisager, pour privilégier un pic prédominant, d'en exciter les différents éléments avec un signal électrique unique couvrant un large spectre de fréquences, et les déphasages appropriés entre les différentes formes d'ondes élastiques individuelles ainsi engendrées pourraient par ailleurs être obtenus par voie acoustique, c'est-à-dire en interposant sur le trajet de chaque faisceau d'ondes élastiques engendrées par le transducteur des blocs de forme convenable d'un matériau transparent auxdites ondes, mais dans lequel elles se propageraient avec une vitesse différente de celle du milieu de transmission au moyen duquel le transducteur est normalement couplé au milieu traité.

La solution décrite est toutefois plus commode à réaliser et permet des réglages faciles.

Aux figures 2 et 3, on a représenté une calotte sphérique en alliage d'aluminium 1, le rayon de la sphère ayant par exemple 40 cm et la hauteur de la calotte étant de 3,8 cm. Sur la surface interne de cette calotte sont montés par exemple 300 éléments piézoélectriques en céramique de forme cylindrique, ayant par exemple 20 mm de diamètre.

Ces éléments sont répartis sur trois cercles concentriques dont deux seulement ont été figurés, dans une zone annulaire qui entoure un transducteur auxiliaire central 2 dont la fonction a été décrite dans le brevet français No 83 20041, déposé le 14 Décembre 1983, au nom du Déposant, pour : "Appareil à impulsions ultrasonores destiné à la destruction des calculs".

On ne décrira ci-après que les particularités qui distinguent l'émetteur de lithotripsie de l'invention de celui dudit brevet.

Les éléments de chacun des jeux 1a-2a..., 1b-2b... de la zone annulaire sont répartis en trois groupes : par exemple les éléments 1a-4a... (3n + 4)a constituent un premier groupe ayant une fréquence de résonance de 300 kHz, les éléments 2a-5a... (3n + 2)a constituent un second groupe ayant une fréquence de résonance de 600 kHz, les éléments 3a-6a... (3n + 3)a, un troisième groupe ayant une fréquence de résonance de 1200 kHz. La distribution des fréquences pour les éléments de rang correspondant des deux autres jeux annulaires est la même. Les fréquences différentes correspondent à des éléments cylindriques de hauteurs différentes ayant des fréquences de résonance respectives F, 2F et 4F, par exemple 10 mm pour 300 kHz, 5 mm pour 600 kHz et 2,5 mm pour 1200 kHz.

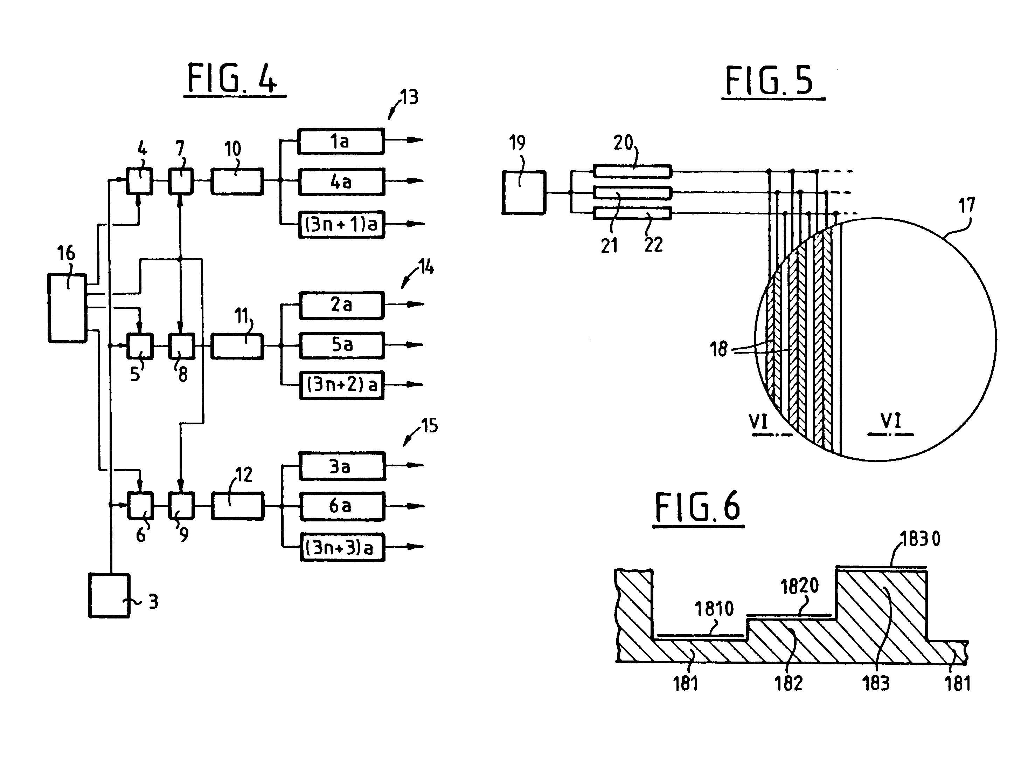

A la figure 4, on a représenté un générateur de synchronisation 3 qui émet des tops dont la durée est de l'ordre de la microseconde et la cadence réglable entre 1/100 et 1 Hz par exemple.

Ces tops sont appliqués, par l'intermédiaire de trois circuits de décalage réglable 4, 5, 6, à trois générateurs 7, 8, 9 d'impulsions à haute fréquence correspondant respectivement aux fréquences de résonance des trois groupes d'éléments, à titre d'exemple 300 kHz, 600 kHz et 1200 kHz respectivement.

Les générateurs 7, 8, 9 attaquent respectivement, par l'intermédiaire d'étages séparateurs respectifs 10, 11, 12, des amplificateurs de puissance 13, 14, 15.

Chaque amplificateur de puissance comporte autant d'étages séparés en parallèle qu'il y a d'éléments dans chaque groupe, pour exciter séparément et en parallèle les différents éléments d'un même groupe.

Les circuits de décalage 4, 5, 6, par exemple réalisés au moyen de lignes à retard, sont réglés pour obtenir une forme d'onde aussi proche que possible de celle qui est souhaitée, conformément aux indications ci-dessus. On a symbolisé en 16 un ordinateur qui effectue, pour commander les réglages, le calcul des formes d'ondes des trois signaux de synchronisation et du signal résultant de leur superposition. Cet ordinateur agira, non seulement sur le réglage des circuits de décalage, mais encore sur ceux des générateurs 7, 8, 9, pour déterminer les trois formes d'ondes et leurs déphasages respectifs.

Un tel dispositif permettrait à l'évidence d'obtenir une grande variété de formes d'ondes du signal acoustique engendré par l'ensemble transducteur, y compris en vue de privilégier par exemple les pics négatifs en vue d'accroître le phénomène de cavitation dans des applications non médicales.

Dans l'application à la lithotripsie plus particulièrement envisagée, le procédé permet, grâce à la réduction considérable de la cavitation, d'effectuer les tirs à des cadences qui pourraient atteindre 150 Hz, en vue de diviser par un facteur de l'ordre de dix les temps de traitement actuels.

A titre de variante du dispositif qui vient d'être décrit, les décalages, obtenus par voie électronique, entre les trois signaux de synchronisation, pourraient être remplacés par des décalages obtenus par l'introduction de différences de longueurs de parcours des signaux acoustiques engendrés par les trois groupes de transducteurs respectifs.

A titre d'exemple, de telles différences peuvent être obtenues en introduisant des cales d'épaisseurs respectives convenables, en matériau apte à transmettre les ultrasons, entre la surface de la calotte 1 et les surfaces arrière d'excitation des éléments piézoélectriques cylindriques des trois groupes.

A la figure 5, on a représenté une sonde destinée à l'échographie et devant par conséquent engendrer des impulsions acoustiques dont le spectre de fréquence soit centré à 5 MHz.

Cette sonde est constituée par un disque 17 en céramique piézoélectrique, ayant par exemple 20 mm de diamètre et sa face rayonnante ou "avant" est plane. Par contre, sa face arrière d'excitation est usinée de manière à former une série de bandes parallèles 18 très fines, ayant par exemple une largeur de 1 mm et des épaisseurs différentes.

On a représenté à la figure 6 une vue agrandie et fragmentaire de l'épaisseur du disque, représentant une succession de groupes de trois bandes telles que 181, 182, 183 ayant respectivement 1/4 mm, 1/2 mm et 1 mm d'épaisseur.

Chacune de ces bandes est munie d'une électrode d'excitation 1810, 1820, 1830 isolée de celle des bandes voisines.

A la figure 5, on a représenté un générateur d'impulsions électriques unique 19 relié aux électrodes respectives 1810, 1820, 1830 par l'intermédiaire de trois lignes à retard réglables 20, 21, 22. Plus précisément, la sortie de chaque ligne à retard est reliée à toutes les électrodes des bandes de même épaisseur des groupes successifs. Bien entendu, les fils de masse, non représentés, sont reliés à une électrode unique, non représentée, fixée à la face avant.

On a ainsi constitué trois ensembles d'éléments transducteurs piézoélectriques ayant des fréquences de résonance respectives de 10 MHz, 5 MHz et 2,5 MHz.

De façon connue en soi, la face arrière du disque sera en outre avantageusement munie d'un bloc amortisseur des oscillations parasites, tandis que la face avant subira un usinage permettant la mise en place de lames d'adaptation d'impédance.

Les retards sont réglés pour correspondre aux décalages souhaités des trois signaux acoustiques de fréquences différentes engendrés par les trois ensembles respectifs, de la manière déjà expliquée. Le signal d'excitation fourni par le générateur 19 doit avoir un spectre de fréquences couvrant les valeurs 10 MHz, 5 MHz et 2,5 MHz.

Ce mode d'exécution est approprié à la réalisation de sondes travaillant à des fréquences élevées (plusieurs mégahertz) : il évite la fabrication délicate d'éléments piézoélectriques individuels indépendants de très petite taille.

Un tel transducteur n'est pas focalisant et devra donc être associé, pour superposer les ondes élastiques individuelles, à un moyen de focalisation, par exemple une lentille acoustique.

## Revendications

1. Générateur d'impulsions élastiques comportant un transducteur piézoélectrique ou magnétostrictif focalisant ou associé à des moyens de focalisation, ledit transducteur ayant une pluralité de surfaces émettrices élémentaires engendrant des signaux élastiques qui se superposent, après propagation sur une certaine distance, dans une région focale et des moyens d'exciter périodiquement à une cadence déterminée plusieurs groupes de surfaces émettrices élémentaires avec des impulsions électriques respectives distinctes, lesdites surfaces émettrices élémentaires appartenant à des éléments transducteurs électriquement isolés les uns des autres (1a, 2a ... ; 1b, 2b... ; respectivement 181, 182, 183) ayant des fonctions de transfert différentes , caractérisé en ce que les fréquences de travail desdits éléments transducteurs sont des multiples entiers de l'une d'entre elles et que des moyens sont prévus pour réaliser entre les signaux élastiques respectifs qui se superposent dans la région focale des déphasages non nuls prédéterminés de façon à obtenir dans ladite région une forme d'onde globale fixée à l'avance.

2. Générateur selon la revendication 1, caractérisé en ce que lesdites fréquences de résonance sont F, 2F, 3F et 4F ou F, 2F et 4F, et les déphasages de chacun des signaux élastiques par rapport à une référence de phase arbitraire commune sont égaux à $-5/(4F_n)$, $F_n$ étant la fréquence de résonance de l'un desdits éléments transducteurs.

3. Générateur selon la revendication 1, caractérisé en ce que les moyens d'excitation comportent des générateurs de fréquences différentes synchronisés par un même signal de synchronisation qui détermine ladite cadence d'émission et des lignes à retard réglables qui constituent lesdits moyens de déphasage.

4. Générateur selon la revendication 1, caractérisé en ce que le transducteur est constitué par un support en forme de calotte sphérique sur la surface de laquelle sont distribués des transducteurs piézoélectriques céramiques élémentaires d'épaisseurs inversement proportionnelles à leurs fréquences de résonance.

5. Générateur selon la revendication 1, caractérisé en ce que lesdits moyens de déphasage sont des éléments aptes à modifier le temps de propagation des ondes élastiques sur ladite distance.

6. Générateur selon la revendication 5, caractérisé en ce que lesdits moyens sont constitués par le décalage des surfaces d'émission des éléments transducteurs les unes par rapport aux autres.

7. Générateur selon la revendication 1, caractérisé en ce que le transducteur est constitué par une céramique monobloc usinée de façon à former des surfaces émissives élémentaires d'épaisseurs différentes électriquement isolées les unes des autres.

## Claims

1. An elastic pulse generator comprising a piezoelectric or magnetostrictive transducer which is focussing or associated with focussing means, said transducer having a plurality of elementary emitting surfaces generating elastic signals which are superimposed, after propagation over a certain distance, in a focal region and means for periodically exciting, at a given rate, several groups of elementary emitting surfaces with respective separate electric pulses, said elementary surfaces belonging to transducer elements electrically isolated from each other (1a, 2a .... 1b, 2b...; respectively 181, 182, 183) having different transfer functions,characterized in that the working frequencies of said transducer elements are whole multiples of one of them and in that means are provided for effecting, between the respective elastic signals which are superimposed in the focal region, non null phase shifts, predetermined so as to obtain in said region a global, previsously established waveform.

2. The generator according to claim 1, characterized in that said resonance frequencies are F, 2F, 3F and 4F or F, 2F and 4F, and that the phase shifts of each of the elastic signals with respect to an arbitrary commun phase reference are equal to $-5/(4F_n)$, $F_n$ being the resonance frequency of one of said transducer elements.

3. The generator as claimed in claim 1, characterized in that the excitation means include generators of different frequencies synchronized by the same synchronization signal which defines said emission rate and adjustable delay lines which form said phase shift means.

4. The generator as claimed in claim 1, characterized in that the transducer is formed by a spherical skull cap shaped support on the surface of which elementary ceramic piezo-electric transducers are distributed whose thicknesses are inversely proportional to their resonance frequencies.

5. The generator as claimed in claim 1, characterized in that said phase shift means are elements adapted for modifying the propagation time of the elastic waves over said distance.

6. The generator as claimed in claim 5, characterized in that said means are formed by the phase shift of the emission surfaces of the transducer elements with respect to each other.

7. The generator as claimed in claim 1, characterized in that the transducer is formed by a one piece ceramic machined so as to form elementary emissive surfaces of different thicknesses electrically isolated from each other.

**Patentansprüche**

1. Generator elektrischer Impulse, mit einem piezoelektrischen oder magnetostriktiven fokussierenden oder Fokussiermitteln zugeordneten Wandler, wobei besagter Wandler eine Vielzahl von elementaren Abgabeflächen aufweist, die elastische Signale erzeugen, welch Signale sich, nach Fortpflanzung über eine bestimmte Entfernung, in einem Fokalbereich überlagern, und mit Mitteln, um periodisch in vorbestimmten Abständen mehrere Gruppen von elementaren Abgabeflächen mit jeweiligen verschiedenen elektrischen Pulsen zu erregen, wobei besagte elementare abgabeflächen zu elektrisch voneinander isolierten Wandlerelementen gehören (1a, 2a ... ; 1b, 2b ... ; bzw. 181,182, 183), die verschiedene Übertragungsfunktionen haben, dadurch gekennzeichnet, dass die Arbeitsfrequenzen der besagten Wandlerelemente ganze Vielfache von einer dieser Frequenzen sind und Mittel vorgesehen sind, um zwischen den jeweiligen elastischen Signalen, die sich im Fokalbereich überlagern, nicht Null gleiche Phasenverschiebungen vorzunehmen, die so vorbestimmt sind, dass man in besagtem Bereich eine im voraus festgelegte globale Wellenform erhält.

2. Generator nach Anspruch 1, dadurch gekennzeichnet, dass besagte Resonanzfrequenzen F, 2F, 3F und 4F sind oder F, 2F und 4F und die Phasenverschiebungen jedes der elektrischen Signale im Verhältnis zu einer gemeinsamen willkürlichen Phasenreferenz gleich $-5/(4F_n)$ sind, wobei $F_n$ die Resonanzfrequenz eines der Wandlerelement ist.

3. Generator nach Anspruch 1, dadurch gekennzeichnet, dass die Erzeugungsmittel Generatoren für verschiedene Frequenzen aufweisen, die durch das gleiche Synchronisiersignal synchronisiert werden, welches die Abgabegeschwindigkeit bestimmt, sowie regelbare Verzögerungslinien, welche die Phasenverschiebungsmittel bilden.

4. Generator nach Anspruch 1, dadurch gekennzeichnet, dass der Wandler von einem Träger in Form einer kugelförmigen Kappe gebildet wird, über dessen Oberfläche piezoelektrische keramische Elementarwandler verteilt sind, deren Dicke entgegengesetzt proportional zu ihren Resonanzfrequenzen ist.

5. Generator nach Anspruch 1, dadurch gekennzeichnet, dass besagte Phasenverschiebungsmittel Elemente sind, welche die Fortpflanzungsdauer der elektrischen Wellen über besagte Entfernung verändern können.

6. Generator nach Anspruch 5, dadurch gekennzeichnet, dass besagte Mittel durch die Verschiebung der Abgabeflächen der Wandlerelemente im Verhältnis zueinander gebildet werden.

7. Generator nach Anspruch 1, dadurch gekennzeichnet, dass der Wandler aus einer einstückigen Keramik besteht, die so bearbeitet wurde, dass sie elektrisch gegeneinander isolierte elementare Abgabeflächen verschiedener Dikke bildet.

EP 0 289 382 B1

FIG.1

a)

b)

FIG.2

FIG.3

8

FIG.5

FIG.6

FIG.4